# EUROPEAN PATENT SPECIFICATION

(11) **EP 3 414 260 B1**
(45) Date of publication and mention of the grant of the patent: **09.10.2019**
(21) Application number: 17723272.5
(22) Date of filing: 12.04.2017
(51) Int. Cl.: C07K 14/54, A61K 38/17, A61K 39/395, C07K 16/28, C07K 16/30

(54) **COMBINATION THERAPY COMPRISING AN INFLAMMATORY IMMUNOCYTOKINE AND A CHIMERIC ANTIGEN RECEPTOR (CAR)-T CELL**
ZUSAMMENSETZUNGSTHERAPIE EINSCHLIESSLICH EIN INFLAMATORISCHES IMMUMOZYTOKIN UND EIN CHIMÄRARTIGES ANTIGENREZEPTOR (CAR)-T-ZELL
THÉRAPIE DE COMBINAISON COMPRENANT UNE CYTOKINE INFLAMATOIRE ET UN RÉCEPTEUR D'ANTIGÈNE CHIMÉRIQUE DIRIGÉ VERS UNE CÉLLULE T

(30) Priority: 12.04.2016 GB 201606181
(43) Date of publication of application: 19.12.2018
(73) Proprietor: Philogen S.p.A., 53100 Siena (IT)
(72) Inventor: BERDEL, Wolfgang, 48149 Muenster (DE); ROSSIG, Claudia, 48149 Muenster (DE); SCHLIEMANN, Christoph, 48151 Muenster (DE); ALTVATER, Bianca, 32457 Porta Westfalica (DE); KAILAYANGIRI, Sareetha, 59065 Hamm (DE)
(74) Representative: Michalski Hüttermann & Partner Patentanwälte mbB
(86) International application number: PCT/EP2017/058873
(87) International publication number: WO 2017/178562

(56) References cited:
- H LODE ET AL.: "Tumor-targeted IL-2 ampliefies T cell-mediated immune response induced by gene therapy with single-chain IL-12", PROCEEDINGS NATIONAL ACADEMY OF SCIENCES PNAS, vol. 96, no. 15, 20 July 1999 (1999-07-20) , pages 8591-8596, XP002224792, National Academy of Sciences ISSN: 0027-8424
- T GARGETT & M P BROWN: "Different cytokine and stimulation conditions influence the expansion and immune phenotype of third-generation chimeric antigen receptor T cells specific for tumor antigen GD2", CYTOTHERAPY, vol. 17, no. 4, January 2015 (2015-01), pages 487-495, XP002760558, ISIS MEDICAL MEDIA, OXFORD, ISSN: 1465-3249
- M G HEGDE ET AL.: "Abstract LB-199:A rationally designed bispecific chimeric antigen receptor molecule that simultaneously redirects T cells to target HER2 and GD2 in osteosarcoma", CANCER RESEARCH , vol. 74, no. 19, Suppl 1 October 2014 (2014-10-01), XP002772085, Proceedings of the 105th Annual Meeting of the American Association for Cancer Research; 2014 Apr 5-9; San Diego, CA. Philadelphia (PA): AACR; DOI: 10.1158/1538-7445.AM2014-LB-199 Retrieved from the Internet: URL:http://cancerres.aacrjournals.org/cont ent/74/19_Supplement/LB-199 [retrieved on 2017-07-13]

### SEQUENCE LISTING

<110> Philogen SPA
<120> Combination therapy comprising an inflammatory immunocytokine and a chimeric antigen receptor (CAR) T-cell
<130> PD43035
<160> 12
<170> PatentIn version 3.5
<210> 1
   <211> 116
   <212> PRT
   <213> Artificial Sequence
<220>
   <223> Vh L19
<400> 1
<210> 2
   <211> 108
   <212> PRT
   <213> Artificial Sequence
<220>
   <223> Vl L19
<400> 2
<210> 3
   <211> 12
   <212> PRT
   <213> Artificial Sequence
<220>
   <223> Linker
<400> 3
   Gly Asp Gly Ser Ser Gly Gly Ser Gly Gly Ala Ser
   1               5                   10
<210> 4
   <211> 133
   <212> PRT
   <213> Homo sapiens
<400> 4
<210> 5
   <211> 17
   <212> **PRT**
   <213> **Artificial** Sequence
<220>
   <223> Fusion protein linker
<400> 5
   Glu Phe Ser Ser Ser Ser Gly Ser Ser Ser Ser Gly Ser Ser Ser Ser
   1               5                   10                  15
   Gly
<210> 6
   <211> 5
   <212> PRT
   <213> Artificial Sequence
<220>
   <223> CDR1 Vh
<400> 6
   Ser Phe Ser Met Ser
   1 5
<210> 7
   <211> 7
   <212> PRT
   <213> Artificial Sequence
<220>
   <223> CDR3 Vh
<400> 7
   Pro Phe Pro Tyr Phe Asp Tyr
   1 5
<210> 8
   <211> 17
   <212> PRT
   <213> Artificial Sequence
<220>
   <223> CDR2 Vh
<400> 8
   Ser Ile Ser Gly Ser Ser Gly Thr Thr Tyr Tyr Ala Asp Ser Val Lys
   1 5 10 15
   Gly
<210> 9
   <211> 12
   <212> PRT
   <213> Artificial Sequence
<220>
   <223> CDR1 Vl
<400> 9
   Arg Ala Ser Gln Ser Val Ser Ser Ser Phe Leu Ala
   1 5 10
<210> 10
   <211> 7
   <212> PRT
   <213> Artificial Sequence
<220>
   <223> CDR2 Vl
<400> 10
   Tyr Ala Ser Ser Arg Ala Thr
   1 5
<210> 11
   <211> 9
   <212> PRT
   <213> Artificial Sequence
<220>
   <223> CDR3 Vl
<400> 11
   Gln Gln Thr Gly Arg Ile Pro Pro Thr
   1               5
<210> 12
   <211> 114
   <212> PRT
   <213> Homo Sapiens
<400> 12
   Asn Trp Val Asn Val Ile Ser Asp Leu Lys Lys Ile Glu Asp Leu Ile
   1               5                   10                  15
   Gln Ser Met His Ile Asp Ala Thr Leu Tyr Thr Glu Ser Asp Val His
               20                  25                  30
   Pro Ser Cys Lys Val Thr Ala Met Lys Cys Phe Leu Leu Glu Leu Gln
           35                  40                  45
   Val Ile Ser Leu Glu Ser Gly Asp Ala Ser Ile His Asp Thr Val Glu
       50                  55                  60
   Asn Leu Ile Ile Leu Ala Asn Asn Ser Leu Ser Ser Asn Gly Asn Val
   65                  70                  75                  80
   Thr Glu Ser Gly Cys Lys Glu Cys Glu Glu Leu Glu Glu Lys Asn Ile
                   85                  90                  95
   Lys Glu Phe Leu Gln Ser Phe Val His Ile Val Gln Met Phe Ile Asn
               100                 105                 110
   Thr Ser

## Description

### Field of the invention

The present invention relates to the field of cellular immunotherapy.

### Background

Cellular immunotherapy of cancer aims to break the tolerance of malignant disease to immune-mediated eradication. One way to achieve this is by adoptive transfer of tumor antigen-specific effector T cells. Major limitations have been the rarity of T cells with specificity and sufficient avidity for tumor-associated antigens within the natural T cell repertoires¹, and the failure of many tumor cells to present antigen to T cells. Chimeric antigen receptor (CAR) engineering now allows to generate large number of tumor-associated antigen-specific T cells. CARs consist of antibody-derived ligand-binding domains linked to stimulatory T cell signaling pathways. Thus they combine antigen recognition and signal transduction in single molecules. CAR engineering can redirect T cells towards tumor surface antigens independent of antigen presentation by MHC complex and thereby overcomes tumor immune escape by down-regulation of MHC-antigen presentation. Indeed, we and others have shown that the interaction of CARs with tumor antigen induces potent T cell effector functions and mediates immunoprotection against tumor growth in murine models.

After 15 years of preclinical and early clinical development, recent results in leukemia have given a substantial boost to the field. CAR-T cell therapies have however also started clinical exploration in non-hematological solid tumors. In a first-in-man clinical phase I/II trial, Louis et al. (2011) have demonstrated moderate antitumor effects of G_{D2}-specific T cells against refractory neuroblastomas that correlated with the *in vivo* persistence of the T cells.

However, no objective responses were reported from further pilot and phase I clinical trials in solid tumors (Kershaw et al, 2006; Lamers et al, 2007; Park et al., 2007). Proc. Natl. Acad. Sci. PNAS 96(15), 20 July 1999, pages 8591-8596 and Cancer Res. 74(19 Suppl. S), 1 October 2014, abstract LB199 disclose a therapy for cancer including immuno-IL2 and a CAR-T cell. Cytotherapy 17(4), January 2015, pages 487-495 discloses the discovery that different cytokines influence the expansion of a chimeric antigen receptor for a CAR-T cell recognizing GD2.

Whereas minimal residual leukemia cells often circulate in peripheral blood, efficient targeting of solid tumors requires the recruitment of CAR T cells to extravascular sites within the tumor. CAR-T cells are most effective at high effector to target cell ratios, while even relatively small tumors with volumes of 1 cm³ can contain over 10⁹ viable cancer cells. For this reason, high numbers of T cells have to infiltrate the tumor. A critical barrier is the tumor microenvironment that protects the tumor cells against immune attack and promotes tumor growth, survival, angiogenesis and invasion. Features of the tumor niche are a lack of immunological danger signals necessary for immune activation, and the presence of immunosuppressive factors and cells with immune-regulatory function.

To become effective, CAR-T cells would have to infiltrate such tumor, and survive and remain functional within this environment, plus efficiently disrupt tumor-induced immunosuppression.

It is one object of the present invention to increase the numbers of intratumoral CAR-T cells and the efficacy of CAR-T cell based therapy in the treatment of medical conditions. It is another object of the present invention to increase the numbers and efficacy of CAR-T cells within tumors for the treatment of solid tumors. It is still another object of the present invention to provide new treatment options in the treatment of neoplastic diseases.

### Embodiments of the invention

These and further objects are met with methods and means according to the independent claims of the present invention. The dependent claims are related to specific embodiments.

### Summary of the invention

Before the invention is described in detail, it is to be understood that this invention is not limited to the particular component parts of the devices described or process steps of the methods described as such devices and methods may vary. It is also to be understood that the terminology used herein is for purposes of describing particular embodiments only, and is not intended to be limiting. It must be noted that, as used in the specification and the appended claims, the singular forms "a," "an" and "the" include singular and/or plural referents unless the context clearly dictates otherwise. It is moreover to be understood that, in case parameter ranges are given which are delimited by numeric values, the ranges are deemed to include these limitation values.

According to one embodiment of the invention, a combination is provided, which combination comprises at least
a) a fusion protein comprising
   a1) a binding protein specifically recognizing a cancer-related antigen and
   a2) an inflammatory cytokine, and
b) a chimeric antigen receptor (CAR)-T cell recognizing a cancer-related antigen,
wherein the binding protein comprises at least one of the group selected from
- antibody,
- modified antibody format,
- antibody derivative or fragment retaining target binding properties
- antibody-based binding protein,
- oligopeptide binder and/or
- an antibody mimetic.

The term "inflammatory cytokine" encompasses a broad and category of small proteins (∼5-30 kDa) which are important in cell signaling and promote systemic inflammation. They are produced predominantly by activated macrophages or lymphocytes and are involved in the upregulation of inflammatory reactions.

The term "binding protein specifically recognizing a cancer-related antigen" refers to proteins or peptides that bind to cancer-related antigens with high specifity and selectivity. In the present context, the binding protein act as homing devices for the cytokine, i.e., directs the cytokine to a tumor site.

The term "cancer related antigen" relates to a structure that (i) can be recognized and bound by a binding protein, e.g., an antibody, with high specificity, sensitivity and affinity, (ii) is highly abundant in precancerous or cancerous tissue, including tumors, lymphoma and leukemia, and (iii) is preferably not abundant, or only lowly abundant, in non-cancerous tissue.

The fusion protein comprising a binding protein and an inflammatory cytokine is called "immunocytokine" hereinafter.

Chimeric antigen T cell receptors are engineered receptors which graft a binding-specificity onto an immune effector T cell, combined with costimulatory domains and the zeta-chain of the T cell receptor for cellular activation after binding. Typically, these receptors are used to graft the specificity of a monoclonal antibody onto a T cell, with transfer of their coding sequence facilitated, e.g., by retroviral vectors.

The most common form of these molecules are fusions of single-chain variable fragments (scFv) derived from monoclonal antibodies, fused to costimulatory domains such as CD28 or 4-1BB and CD3-zeta transmembrane and endodomain. Such molecules result in the effective transmission of a zeta signal in response to binding by the scFv of its target.

The inventors have surprisingly shown that an immunocytokine which binds to a tumor-specific target dramatically increases infiltration of CAR-T cells into the respective tumor. Without being bound to theory, tumor infiltration of CAR-T cells seems to be a very limited.

In addition, the combination of naked immunocytokines with non-transduced T-cells also yields only limited tumor infiltration of these cells. The finding that both signals, via CAR and an immunocytokine, cause effective infiltration, is a surprising fact.

Different speculations exist to explain this finding. Active tumor-mediated immunosuppression may have a role in limiting the efficacy of CAR-T cells (Zou 2005), while other authors blame functional changes in T lymphocytes after their *ex vivo* manipulation for the reduced ability of cultured CAR-T cells to penetrate tumors (Caruana et al, 2015). It also appears that tumors are oftentimes surrounded by a desmoplastic stroma that the cells need to penetrate.

None of these theories, however, plausibly suggests that a combination of CAR-T cells with an immunocytokine would improve tumor infiltration of the cells. There is no plausible rationale that could explain how the immunocytokine would overcome the problems discussed above with respect to tumor infiltration of CAR-T cells

Based on the current knowledge, it was therefore surprising to find that the addition of such fusion protein to suitable CAR-T cells so dramatically enhances the tumor infiltration of the cells, hence allowing an increase of anti-tumor efficacy.

Prior art teaches away from such solution. WO2015164354A1 discloses CAR-T cell therapy (in particular CD 19 CARs) in combination with a IL-33 pathway inhibitor. The rationale behind this combination is that some CAR-T cells were suspected to caused cytokine-related disease ("cytokine storms"). To avoid or ameliorate this consequence, the authors suggest coadministration of an IL-33 pathway inhibitor.

Pegram et al. (2015) suggest CD-19 CAR-T cells with transgenic IL-12 (CD 19 CARs (19z1IRESIL-12). Such cells express IL-12 and allegedly increase anti-tumor efficacy. The authors explain that the systemic administration of IL-12, which has been done in a parallel experiment, would cause inflammatory side effects (cytokine storm), hence their embodiment would be advantageous. However, because IL12 is expressed *in situ* by the CAR-T cells, the dosing thereof cannot be controlled, which generates substantial risks. Further, said embodiment cannot enhance penetration of the CAR-T cells into the tumor.

According to one embodiment, the cancer-related antigen recognized by the binding protein is cancer stroma related, and/or the chimeric antigen receptor (CAR)-T cell recognizes a cancer-cell related antigen.

Such embodiment relies on a defined interplay between the binding protein and the CAR-T cell, with the former binding to cancer stroma and the latter binding to cancer cells. Without being bound to theory, this combination provides the advantage that binding protein and CAR-T cells do not compete for the same targets (e.g., antigens), so as to ensure that each can find its suitable target.

On the other hand, this approach relies on the assumption that the cancer stroma related antigen and the cancer-cell related antigen are expressed in the same tumor. This is not necessarily the case.

Because the binding protein (or the immunocytokine, to be precise) alone has no cytotoxic activity, it does not necessarily have to bind to cancer cells. The CAR-T cells by contrast, do actually have to bind to cancer cells, to exert their cell killing effect.

The term "cancer stroma related antigen" relates to a structure that (i) can be recognized and bound by a binding protein, e.g., an antibody, with high specificity, sensitivity and affinity, (ii) is highly abundant in the cancer stroma, i.e., the microenvironment surrounding the tumor cells. One example of such cancer stroma related antigen is an antigen that occurs on Cancer-associated fibroblasts (CAFs), which in some tumors make up the bulk of cancer stroma and affect the tumor microenvironment such that they promote cancer initiation, angiogenesis, invasion, and metastasis.

The term "cancer cell related antigen" relates to a structure that (i) can be recognized and bound by a binding protein, e.g., an antibody, with high specificity, sensitivity and affinity, (ii) is highly abundant on the cellular surface of precancerous or cancerous cells, including tumors, lymphoma and leukemia, and (iii) is preferably not abundant, or only lowly abundant, in non-cancerous tissue.

In one embodiment of the invention, the cancer-related antigen recognized by the binding protein is an angiogenesis marker.

Angiogenesis markers are proteins that are primarily expressed during angiogenesis, i.e., the process in which new blood vessels form from pre-existing vessels. Angiogenesis is a fundamental step in the transition of tumors and lymphomas from a benign state to a malignant one, because the rapidly proliferation of cancer tissue develops a high demand for nutrients and oxygen as well as export of metabolic waste products, which requires thorough vascularization. Hence, the respective markers are suitable to target cancer specific structures which are related to the vascularization of tumors, and cancer tissues in general.

In one further embodiment of the invention, the cancer related antigen recognized by the binding protein is a fibronectin, or a spliced isoform thereof, of a subdomain thereof.

Fibronectin is a high-molecular weight (∼440kDa) glycoprotein of the extracellular matrix that binds to membrane-spanning receptor proteins called integrins. Fibronectin binds extracellular matrix components such as collagen, fibrin, and heparan sulfate proteoglycans. Fibronectin exists as a protein dimer, consisting of two nearly identical monomers linked by a pair of disulfide bonds. The fibronectin protein is produced from a single gene, but alternative splicing of its pre-mRNA leads to the creation of at least 20 different isoforms in humans, the functions of which are discussed, inter alia, in White and Muro 2011.

In one particular embodiment of the invention, the cancer-related antigen recognized by the binding protein is a splice isoform of fibronectin. In one other particular embodiment such splice isoform of fibronectin is the ED-B domain

The term "ED_{B} domain", or "ED-B-domain", is to be understood as the extra-domain B of human fibronectin. It is often referred to as EDB, EIIIB or EDII.

The extra domain B (ED_{B}) of fibronectin is one of the best-characterized markers of angiogenesis described so far (Zardi et al., 1987; Kaspar et al. 2006). This 91-amino acid type III homology domain can be inserted into the fibronectin molecule during active tissue remodeling by a mechanism of alternative splicing (Zardi et al., supra). ED_{B} is essentially undetectable in healthy adult tissues but is highly abundant in the vasculature of many aggressive solid tumors, in particular in the stroma thereof, thus making ED_{B} a suitable target for anti-cancer therapy as suggested herein. Anti-ED_{B}-antibodies are known in the prior art, and are e.g. described in WO 97/45544.

Preferably, the binding protein which binds to the ED_{B}-domain of fibronectin exhibits a high affinity for the ED_{B}-domain of FN. In particular, the binding protein binds to the ED_{B} fibronectin domain with nanomolar or subnanomolar affinity. Such binding proteins are known in the prior art and are e.g. described in WO99/58570.

In one specific embodiment, the binding protein specifically binds to the ED_{B} oncofetal fibronectin domain. One such binding protein is huBC1, which is a humanized antibody that targets a cryptic sequence of the human ED-B-containing fibronectin isoform, B-FN, present in the subendothelial extracellular matrix of most aggressive tumors. B-FN is oncofetal and angiogenesis-associated.

In one further embodiment of the invention, the inflammatory cytokine is one selected from the group consisting of IL2 and IL15.

IL2 and IL15 belong to the common γ-chain family of cytokines. Interleukin 2 (IL2) is a cytokine signaling molecule of the immune system which regulates the activities of white blood cells that are responsible for immunity. IL2 mediates its effects by binding to IL2 receptors, which are expressed by lymphocytes. IL2 has a direct effect on T cells, in that it promotes the differentiation of T cells into effector T cells and into memory T cells when the initial T cell is also stimulated by an antigen.

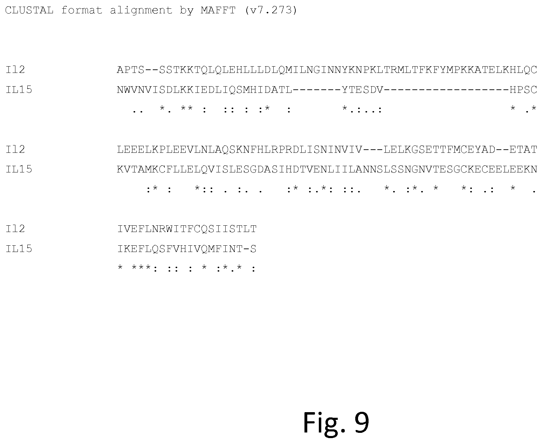

Interleukin 15 (IL15) is a cytokine with structural similarity to IL2 (see Fig. 9). Like IL2, IL15 binds to and signals through a complex composed of IL2/IL15 receptor beta chain (CD122) and the common gamma chain (gamma-C, CD132). As a consequence, the two cytokines share signaling elements and functions, specifically induction of T cell proliferation. IL15 is secreted by mononuclear phagocytes e.g. following infection by virus(es). It has a key role in maintaining populations of memory T cells over long periods of time by homeostatic expansion.

In one embodiment of the invention, the CAR-T cell recognizes disialoganglioside GD2. GD2 is a disialoganglioside antigen expressed on tumor cells of neuroectodermal origin, including human neuroblastoma, Ewing sarcoma and melanoma, with highly restricted expression on normal tissues, principally to the cerebellum and peripheral nerves in humans. It is hence a suitable target for therapeutic approaches with monoclonal antibodies or CAR-T cells.

"Antibodies", also synonymously called "immunoglobulins" (Ig), are generally comprising four polypeptide chains, two heavy (H) chains and two light (L) chains, and are therefore multimeric proteins, or an equivalent Ig homologue thereof (e.g., a camelid nanobody, which comprises only a heavy chain, single domain antibodies (dAbs) which can be either be derived from a heavy or light chain); including full length functional mutants, variants, or derivatives thereof (including, but not limited to, murine, chimeric, humanized and fully human antibodies, which retain the essential epitope binding features of an Ig molecule, and including dual specific, bispecific, multispecific, and dual variable domain immunoglobulins; Immunoglobulin molecules can be of any class (e.g., IgG, IgE, IgM, IgD, IgA, and IgY), or subclass (e.g., IgG1, IgG2, IgG3, IgG4, IgA1, and IgA2) and allotype.

An "antibody-based binding protein", as used herein, may represent any protein that contains at least one antibody-derived V_{H}, V_{L}, or C_{H} immunoglobulin domain in the context of other non-immunoglobulin, or non-antibody derived components. Such antibody-based proteins include, but are not limited to (i) F_{c}-fusion proteins of binding proteins, including receptors or receptor components with all or parts of the immunoglobulin C_{H} domains, (ii) binding proteins, in which V_{H} and or V_{L} domains are coupled to alternative molecular scaffolds, or (iii) molecules, in which immunoglobulin V_{H}, and/or V_{L}, and/or C_{H} domains are combined and/or assembled in a fashion not normally found in naturally occurring antibodies or antibody fragments.

An "antibody derivative or fragment", as used herein, relates to a molecule comprising at least one polypeptide chain derived from an antibody that is not full length, including, but not limited to (i) a Fab fragment, which is a monovalent fragment consisting of the variable light (V_{L}), variable heavy (V_{H}), constant light (C_{L}) and constant heavy 1 (C_{H}1) domains; (ii) a F(ab')2 fragment, which is a bivalent fragment comprising two Fab fragments linked by a disulfide bridge at the hinge region; (iii) a heavy chain portion of a F_{ab} (F_{d}) fragment, which consists of the V_{H} and C_{H}1 domains; (iv) a variable fragment (Fᵥ) fragment, which consists of the V_{L} and V_{H} domains of a single arm of an antibody, (v) a domain antibody (dAb) fragment, which comprises a single variable domain; (vi) an isolated complementarity determining region (CDR); (vii) a single chain Fᵥ Fragment (scFᵥ); (viii) a diabody, which is a bivalent, bispecific antibody in which V_{H} and V_{L} domains are expressed on a single polypeptide chain, but using a linker that is too short to allow for pairing between the two domains on the same chain, thereby forcing the domains to pair with the complementarity domains of another chain and creating two antigen binding sites; and (ix) a linear antibody, which comprises a pair of tandem Fᵥ segments (V_{H}-C_{H}1-V_{H}-C_{H}1) which, together with complementarity light chain polypeptides, form a pair of antigen binding regions; and (x) other non-full length portions of immunoglobulin heavy and/or light chains, or mutants, variants, or derivatives thereof, alone or in any combination. In any case, said derivative or fragment retains target binding properties

The term "modified antibody format", as used herein, encompasses antibody-drug-conjugates, Polyalkylene oxide-modified scFv, Monobodies, Diabodies, Camelid Antibodies, Domain Antibodies, bi- or trispecific antibodies, IgA, or two IgG structures joined by a J chain and a secretory component, shark antibodies, new world primate framework + non-new world primate CDR, IgG4 antibodies with hinge region removed, IgG with two additional binding sites engineered into the CH3 domains, antibodies with altered Fc region to enhance affinity for Fc gamma receptors, dimerised constructs comprising CH3+VL+VH, and the like.

The term "antibody mimetic", as used herein, refers to proteins not belonging to the immunoglobulin family, and even non-proteins such as aptamers, or synthetic polymers. Some types have an antibody-like beta-sheet structure. Potential advantages of "antibody mimetics" or "alternative scaffolds" over antibodies are better solubility, higher tissue penetration, higher stability towards heat and enzymes, and comparatively low production costs.

Some antibody mimetics can be provided in large libraries, which offer specific binding candidates against every conceivable target. Just like with antibodies, target specific antibody mimetics can be developed by use of High Throughput Screening (HTS) technologies as well as with established display technologies, just like phage display, bacterial display, yeast or mammalian display. Currently developed antibody mimetics encompass, for example, ankyrin repeat proteins (called DARPins), C-type lectins, A-domain proteins of S. aureus, transferrins, lipocalins, 10th type III domains of fibronectin, Kunitz domain protease inhibitors, ubiquitin derived binders (called affilins), gamma crystallin derived binders, cysteine knots or knottins, thioredoxin A scaffold based binders, SH-3 domains, stradobodies, "A domains" of membrane receptors stabilised by disulfide bonds and Ca2+, CTLA4-based compounds, Fyn SH3, and aptamers (peptide molecules that bind to a specific target molecules).

In one particular embodiment of the invention, the binding protein contains at least one CDR sequence of the L19 antibody. The tumor-targeting ability of the high-affinity human antibody L19 (Pini et al., 1998), specific to ED_{B}, has been well established both in animal models of cancer (Borsi et al., 2002; Berndorff et al., 2006; Berndorff et al., 2005; Demartis et al., 2001) and in patients with solid tumors (Santimaria et al., 2003). Recently, ED_{B} expression was also found in the majority of lymphoma-infiltrated tissue samples from various Non-Hodgkin lymphoma patients (Sauer et al., 2006), as well as in Hodgkin lymphoma (Schliemann et al, 2009).

The binding protein specifically recognizing ED_{B} fibronectin, in particular the L19 antibody, can be employed in various antibody formats. Preferred antibody formats are full IgG, Fab, (Fab')₂, scFv, diabody, or minibody format. Especially preferred are the full IgG, scFv and SIP format for the L19 antibody. Most preferred is the L19 antibody in the scFv format. Several immunoprotein formats are known in the prior art, e.g. based on the CH3 domain or the εₛ₂-CH4 domain of IgE. The preferred SIP format for L19 based on the εₛ₂-CH4 domain of IgE and L19 in full IgG format are for example described in WO03/076469.

In one embodiment of the invention, the binding protein comprises the sequences according to SEQ ID No. 6 to 11. Preferably, the binding protein comprises at least one V heavy chain according to SEQ ID No. 1 or at least one V light chain according to SEQ ID No. 2.

In one further embodiment of the invention, the heavy and the light chain are connected by a peptide linker.

In one preferred embodiment of the invention, the peptide linker comprises a sequence according to SEQ ID No 3, or a sequence having at least 90% identity to the sequence according to SEQ ID No 3.

In another embodiment of the invention, the IL2 or the IL15 is mammalian IL2 or IL15, preferably human IL2 or IL15, or a functional variant thereof.

Functional variants of IL2 or IL15 are variants of human IL2 or IL15 which exhibit at least 10%, but more preferably more than 50%, and even more preferred more than 90% of the activity of native human IL2 or IL15. Interleukin activities are activities of Interleukin in biochemical assays or *in vivo.*

IL2 activity can be measured by the effect on proliferation and/or differentiation of activated T and B lymphocytes and of natural killer cells and/or induction of cytotoxic T cell activity and/or NK/lymphokine activated killer (LAK) anti-tumor activity (Meazza et al., 1996).

In particular, functional variants are cystein-125 muteins of Interleukin 2 as described in EP0109748 and other muteins, including cystein muteins as described in EP0136489, in particular serine 125-Interleukin 2. Also, the N-terminus of hIL2 variants may be altered without significantly affecting the activity, in particular the N-terminal 1-5 amino acids, especially preferred the N-terminal Alanine may be deleted or altered, preferably deleted. Moreover, the Interleukin 2 may contain altered or deleted post-translational modifications, in particular the glycosylation pattern may be altered or missing. Different or absent glycosylation may be obtained e.g. either by mutating the sequence or by expression of the fusion protein in an appropriate host. For example, Aldesleukin, which is approved for metastatic RCC, is unglycosylated des-alanyl-1, serine-125 human interleukine-2 produced in *E. coli.*

Interleukin 15 activity can be determined with the methods disclosed by Paxton 2001. Functional variants of IL15 are, inter alia, ALT-803, produced by Alter Bioscience; which is a combined IL15N72D mutant and the soluble domain of IL15Rα. As of 2014 INDs have been submitted for clinical trials for 4 indications: metastatic melanoma, relapse of hematological malignancies after allogeneic stem cell transplantation, refractory multiple myeloma, and BCG-naïve non-muscle invasive bladder cancer in combination with BCG.

Both Interleukin 2 and Interleukin 15 may be produced recombinantly or may be isolated from mammalian or human tissue.

In one particular embodiment of the invention, the IL2 comprises a sequence according to SEQ ID No 4, or a functional variant thereof.

Said sequence of human Interleukin 2 after cleavage of the propeptide has 133 AA residues, while the precursor comprising the propetide has 153 AA residues

In another particular embodiment of the invention the IL15 comprises a sequence according to SEQ ID No 12, or a functional variant thereof.

Said sequence of human Interleukin 15 after cleavage of the propeptide has 114 AA residues, while the precursor comprising the propeptide has 133 AA residues.

The binding protein and the cytokine may be fused directly to one another, or by means of one or more chemical linkers or peptide linkers. Such fusion proteins are known in the prior art and are e.g. described in WO01/062298.

In one embodiment of the invention a fusion protein linker is connecting the binding protein and the inflammatory cytokine part. Preferably, the fusion protein linker has a length of between ≥ 1 and ≤ 30 amino acids.

In one preferred embodiment, the fusion protein linker comprises a sequence according to SEQ ID No. 5.

The fusion protein may be monomeric, or multimeric, e.g., dimeric. Dimeric or other multimeric forms may be formed covalently or non-covalently. The fusion proteins are preferably produced recombinantly using methods known to the skilled person. In particular, prokaryotic or eukaryotic expression systems, e.g. yeast or mammalian expression systems, can be used.

Preferably, the fusion protein is the L19-IL2 conjugate Darleukin, manufactured by Philogen S.p.A. Darleukin is disclosed, inter alia, in List and Neri (2013).

In another embodiment, the fusion protein is an L19-IL15 conjugate, manufactured by Philogen S.p.A., and disclosed, inter alia, in Kaspar et al 2007.

In one particular embodiment of the invention, the chimeric antigen receptor (CAR) in the T-cell comprises 14.G2a-zeta, 14.G2a-BBzeta or 14.G2a-28zeta.

14.G2a-zeta is a fusion of a scFv derived from hybridoma 14g2a, which recognizes disialoganglioside GD2. 14.G2a is a GD2-specific antibody from which the CAR was derived. The hybridoma cell line 14.G2a (mouse IgG2a;κ) 15 was generated by Dr. R.A. Reisfeld (La Jolla, CA) (Mujoo et al., 1989).

14.G2a-BBzeta is a 2^{nd} generation CAR which furthermore comprises 4-1BB (CD137), which acts as the costimulatory signaling domain of the CAR, and serves to enhance antigen activation and increase potency (Imai et al., 2004). 14.G2a-BBzeta and GD2.BBz are used interchangeably herein.

The alternative 2nd generation CAR, 14.G2a-28zeta, alternatively designated GD2.28z, (Liebsch et al. Br J Cancer 2014. PMID: 23839490) contains the costimulatory domain of CD28, also to increase CAR-mediated T cell activation.

According to another aspect of the invention, the combination according to the above description for use in the treatment of a human or animal subject
- suffering from,
- at risk of developing, and/or
- being diagnosed for
a given pathologic condition is provided. Preferably, said pathologic condition is a neoplastic disease. The term neoplastic disease refers to any abnormal growth of tissues or cells, in particular of malignant growth. It encompasses primary cancers, secondary cancers and metastases, including carcinoma, sarcoma, melanoma, lymphoma, and leukemia.

In a preferred embodiment, the pathologic condition is a solid tumor, in particular a lymphoma, carcinoma, or a sarcoma. In another preferred embodiment the pathologic condition is leukemia.

According to another aspect of the invention, the fusion protein and the chimeric antigen receptor (CAR)-T cell are to be administered as concomitant and/or adjunctive therapy.

Adjunctive therapy is therapy that is given in addition to the primary, main, or initial therapy to maximize its effectiveness. Concomitant therapy refers to administering a given medical treatments at the same time as another treatment.

According to another aspect of the invention, the fusion protein and the chimeric antigen receptor (CAR)-T cell are to be administered as sequential therapy. For example, in one embodiment the human or animal patient is first treated with the fusion protein, and then with the CAR-T cells. In other embodiments, an alternating administration scheme can be used.

### Experiments and Figures

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

### Figures

Fig. 1: Experimental design for assessing the antitumor activity of L19-IL2 and CAR-T cells cotargeting against localized Ewing sarcoma xenografts. The following therapy groups were used:

| **run** | **antibody** | **T cells** |
|---|---|---|
| 1 | KSF-IL2 | |
| 2 | L19-IL2 | |
| 3 | | non-transduced T cells |
| 4 | | CAR-T (14.G2a-BBzeta) |
| 5 | L19-IL2 | non-transduced T cells |
| 6 | L19-IL2 | CAR-T (14.G2a-BBzeta) |

Figs. 2 - 5 show the results of T cell infiltration experiments by CD3 staining.
Fig. 2A: KSF-IL2; Fig. 2B: L19-IL2. No particular CAR-T cell infiltration can be detected. Fig. 3A: Irrelevant, non-transduced T cells without L19-IL2. Intratumoral T cell infiltration of about 1% can be detected. The quantitative estimation of percentages relies on a rough estimation according to routine of an experienced pathologist.
Fig. 3B: CAR-T cells (14.G2a-BBzeta, but without L19-IL2): Intratumoral T cell infiltration is about 5%, but no intravascular and no peritumoral T cells can be found
Fig. 4: Non-transduced T cells plus L19-IL2. Intratumoral T cell infiltration is about 3% but most of the T cells were found to be peritumoral
Fig. 5: CAR-T cells (14.G2a-BBzeta) + L19-IL2. Intratumoral T cell infiltration is about 10%, intravascular: T cell infiltration is about 30%, Peritumoral T cell infiltration is about 60%.

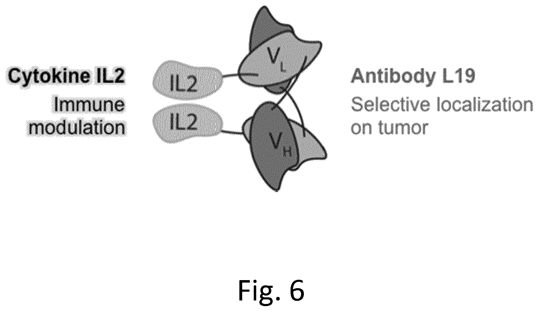

Fig. 6: Schematical drawing of the immunocytokine Darleukin (L19-IL2). Note that a preferred version of the L19-IL15 conjugate discussed herein has a similar shape.

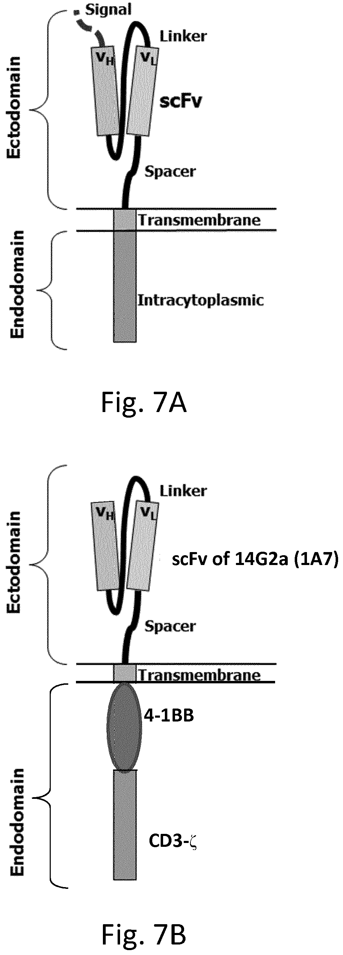

Fig. 7A: Different components of an exemplary 1^{st} generation chimeric antigen receptor. In this example, the artificial TCR comprises a fusion of an antibody component, e.g., a single-chain variable fragment (scFv) derived from a given monoclonal antibody, fused to the CD3-zeta transmembrane and endodomain. Such molecules transmit a zeta signal in response to target binding of the antibody component. When T cells express this molecule (usually achieved by oncoretroviral vector transduction), they recognize and kill target cells that express the target detected by the antibody component.
Fig. 7B: Schematic structure of the chimeric antigen receptor 14.G2a-BBzeta (2^{nd} generation). The construct comprises an scFv fragment of the antibody 14G2a (1A7), fused to the 4-1BB domain and the CD3-zeta (CD3ζ) domain by means of suitable spacers or linkers. The CD3ζ domain transmits a proliferative signal upon binding of the scFv fragment to its target, GD2.The 4-1BB costimulatory signaling domain mimic amplifies the activation of the CAR-T cells, leading to a more robust signal to the T cell to multiply and kill the cancer cell.

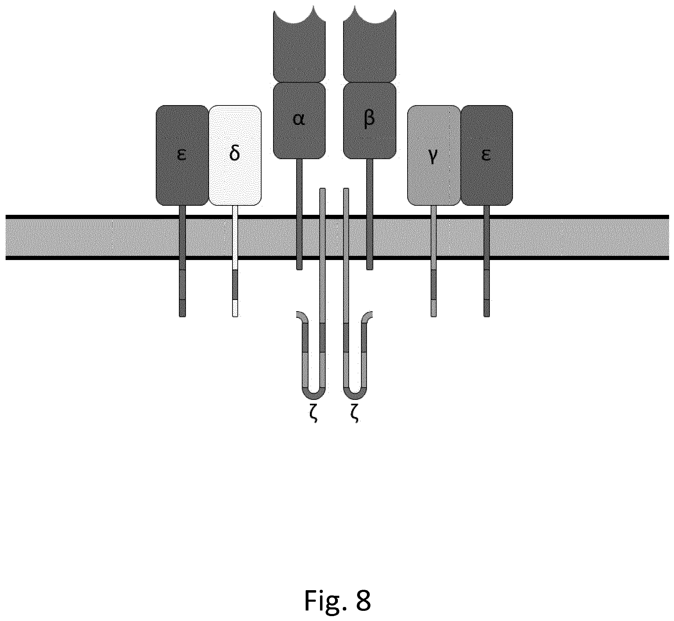

Fig. 8: The T-cell receptor complex. CD3-zeta is a chain of the CD3 T-cell co-receptor, which comprises a CD3γ chain, a CD3δ chain, and two CD3ε chains. These chains associate with TCR-α and TCR-β chains and the CD3ζ-chain (zeta-chain) to generate an activation signal in T lymphocytes. The TCR, CD3ζ-chain, and CD3 molecules together constitute the TCR complex.
Fig. 9: Sequence alignment between IL2 and IL15. Note the structural similarity between the two cytokines.

### Materials and Methods

### 1. Sarcoma xenograft experiments

A localized Ewing sarcoma model which relies on subcutaneous xenografting of 2x10⁶ VH-64 Ewing sarcoma cells per mouse into NOD/scid gamma (NSG) mice was produced.

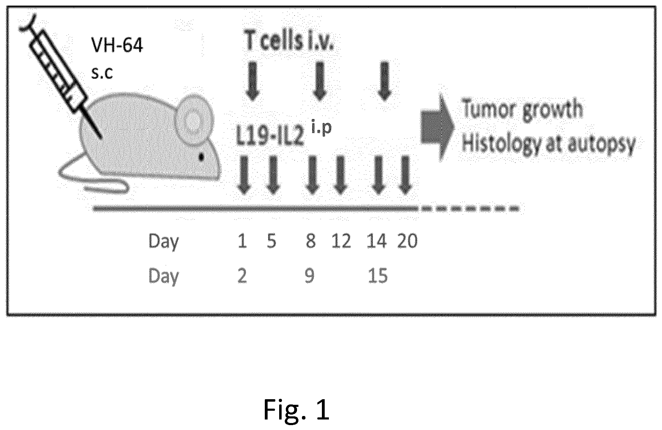

Upon a tumor volume of 200-300 mm³ mice received intraperitoneal treatment with L19-IL2 (30 µg twice-weekly on days 1, 5, 8, 12, 14, and 20), and with intravenous injection of 3 doses of 1x10⁷ 14.G2a-BBzeta-transduced T cells, or non-transduced T cells as controls (see Fig. 1). Tumor growth was monitored by caliper quantification of diameters. 2 mice were used in each cohort. Post-therapy tumor sections were used for comparative histopathological analysis with regard to (CAR)-T cell infiltration and immunocytokine localization. Furthermore, localization of L19-IL2 within the tumor tissue was evaluated using an antihuman IL2 antibody in standard immunofluorescence procedures. L19-IL2 and 14.G2a-BBzeta are described in details elsewhere herein. Control experiments were done with
(1) L19-IL2 or 14.G2a-BBzeta, respectively, alone
(2) KSF-IL2, which is an immunoconjugate binding to hen egg lysozyme (KSF), and serves as negative control
(3) non-transduced T cells likewise serve as negative controls.

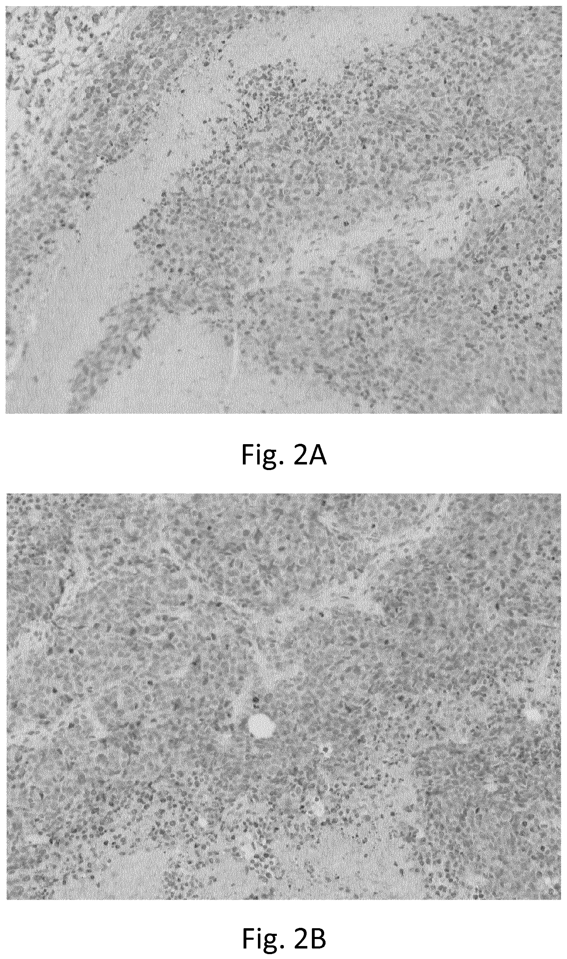

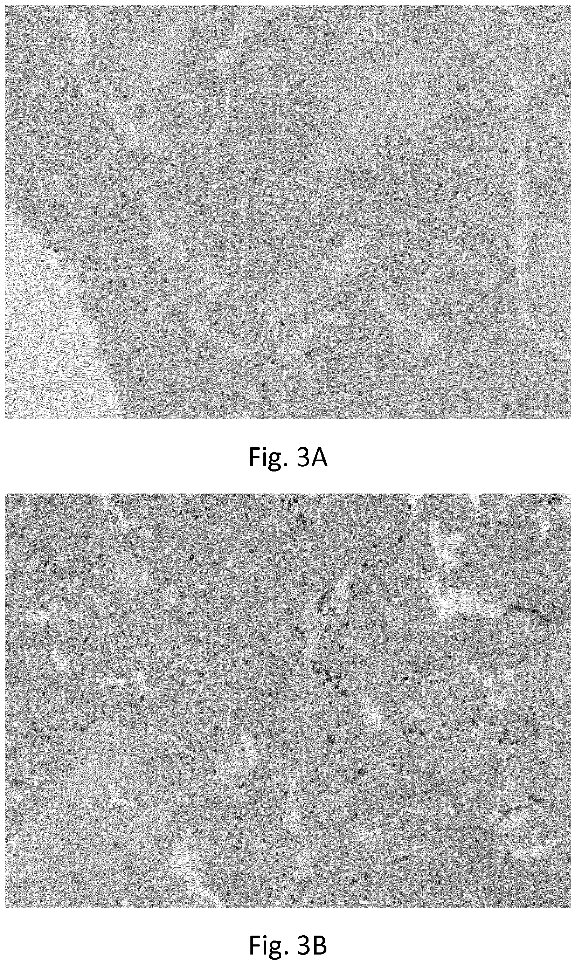

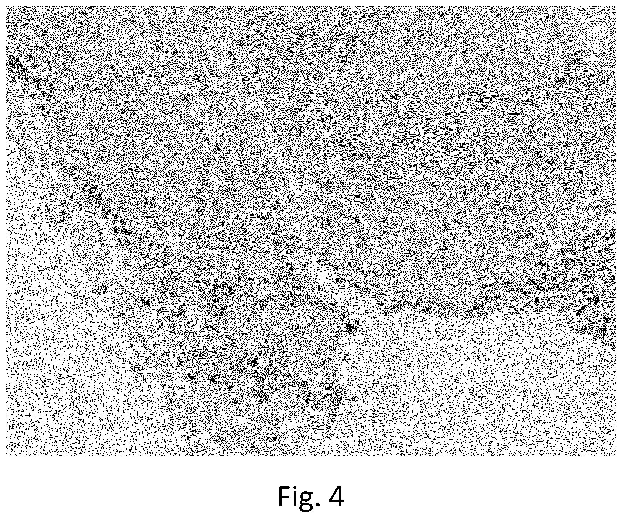

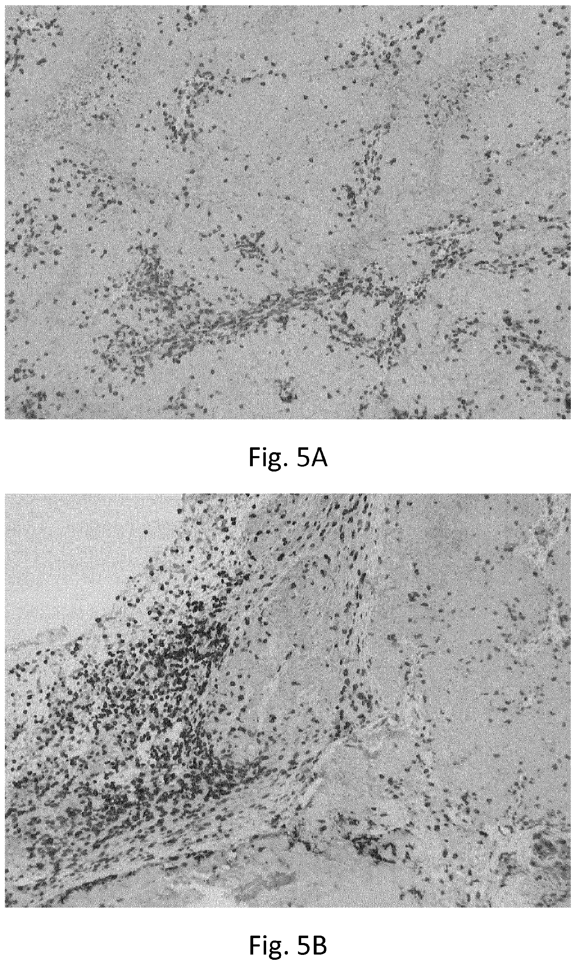

Results of this experiment are shown in Figs 2 - 5.

The combination of CAR-T cells and the immunocytokine drastically increased tumor infiltration - a finding which was completely unanticipated, because none of the current theories that explain the challenges CAR-T cells face when infiltration a solid tumor (active tumor-mediated immunosuppression, functional changes in T lymphocytes after *ex vivo* manipulation, physical inhibition of infiltration by the desmoplastic stroma which the cells need to penetrate) would render the synergistic effect the immunocytokine has on CAR-T cell infiltration obvious.

The functional implication of a cytokine, namely to merely regulate the activity of T cells, can not explain its supportive effect in the present scenario, where tumor-mediated immunosuppression, functional changes in T lymphocytes after *ex vivo* manipulation and/or physical inhibition of infiltration by the desmoplastic stroma challenge the anti tumor efficacy of the T cells.

### References

Kowalczyk A et al. (2009), Cancer letters vol. 281 (2) p. 171-82
List T, Neri D (2013), Clinical pharmacology: advances and applications vol. 5 p. 29-45
Imai C et al. (2004), Leukemia, Apr;18(4):676-84
Zou W (2005) Nat. Rev. Cancer 5, 263-274
Caruana I et al. (2015), Nature medicine vol. 21 (5) p. 524-9
Louis C U et al. (2011), Blood, 118: 6050-6
Kershaw M H et al (2005), Clin Cancer Res, 12: 6106-6115
Lamers C H J et al. (2007), Cancer Immunol Immunother, 56: 1875-1883
Park J R et al. (2007), Molecular Therapy 15: 825-833
Schliemann C et al. (2009), Leuk Res. Dec;33(12):1718-22
Zardi et al. (1987), Embo J.;6:2337-2342
Kaspar et al. (2006), Int J Cancer, 118:1331-1339
Pini et al. (1998), J Biol Chem.;273:21769-21776
Borsi et al. (2002), Int J Cancer.;102:75-85
Berndorff et al. (2006), J Nucl Med.;47:1707-1716
Berndorff et al. (2005), Clin Cancer Res.;11:7053s-7063s
Demartis et al. (2001), Eur J Nucl Med;28:534-53
Meazza et al. (1996), Br.J.Cancer. 74:788-795
White ES, Muro AF (2011), IUBMB life vol. 63 (7) p. 538-46
Rybak et al (2007), Cancer research vol. 67 (22) p. 10948-57
Paxton, R J (2001), Current protocols in immunology / edited by John E. Coligan ... [et al.] vol. Chapter 6 p. Unit 6.22
Kaspar et al (2006), Cancer research vol. 67 (10) p. 4940-8
Pegram et al. (2015), Leukemia vol. 29 (2) p. 415-22
Mujoo, K; Kipps, T J; Yang, H M; Cheresh, D A; Wargalla, U et al. (1989), Cancer research,vol. 49 (11) p. 2857-61

**Sequence Listing**

| **Seq No** | **Specification** | **Sequence (One letter code)** |
|---|---|---|
| 1 | Vh L19 | |
| 2 | Vl L19 | |
| 3 | scFv Linker | GDGSSGGSGGAS |
| 4 | human IL2 | |
| 5 | Fusion protein linker | EFSSSSGSSSSGSSSSG |
| 6 | CDR1 Vh | SFSMS |
| 7 | CDR3 Vh | PFPYFDY |
| 8 | CDR2 Vh | SISGSSGTTYYADSVKG |
| 9 | CDR1 Vl | RASQSVSSSFLA |
| 10 | CDR2 Vl | YASSRAT |
| 11 | CDR3 Vl | QQTGRIPPT |
| 12 | human IL15 | |

## Claims

1. A combination comprising at least
a) a fusion protein comprising
a1) a binding protein specifically recognizing a cancer-related antigen and
a2) an inflammatory cytokine, and
b) a chimeric antigen receptor (CAR)-T cell recognizing a cancer-related antigen,
wherein the binding protein comprises at least one of the group selected from
• antibody,
• modified antibody format,
• antibody derivative or fragment retaining target binding properties
• antibody-based binding protein,
• oligopeptide binder and/or
• an antibody mimetic.

2. The combination according to claim 1, wherein
• the cancer-related antigen recognized by the binding protein is cancer stroma-related and/or
• the chimeric antigen receptor (CAR)-T cell recognizes a cancer cell-related antigen.

3. The combination according to claim 1, wherein the cancer-related antigen recognized by the binding protein is an angiogenesis marker.

4. The combination according to any of the aforementioned claims, wherein the cancer-related antigen recognized by the binding protein is a fibronectin, or a splice isoform thereof, and/or a subdomain thereof.

5. The combination according to any of the aforementioned claims, wherein the cancer-related antigen recognized by the binding protein is the ED_{B.}domain of fibronectin.

6. The combination according to any of the aforementioned claims, wherein the inflammatory cytokine is one selected from the group consisting of IL2 and IL15.

7. The combination according to any of the aforementioned claims, wherein the CAR-T cell recognizes disialoganglioside GD2.

8. The combination according to any of the aforementioned claims, wherein the binding protein contains at least one CDR sequence of the L19 antibody.

9. The combination according to any of the aforementioned claims, wherein the binding protein comprises the sequences according to SEQ ID No. 6 to 11.

10. The combination according to any of the aforementioned claims, wherein the binding protein comprises at least one V heavy chain according to SEQ ID No. 1 or at least one V light chain according to SEQ ID No. 2.

11. The combination according to any of the aforementioned claims, wherein the heavy and the light chain are connected by a peptide linker, wherein preferably the peptide linker comprises a sequence according to SEQ ID No 3, or a sequence having at least 90% identity to the sequence according to SEQ ID No 3.

12. The combination according to any of the aforementioned claims, wherein the IL2 or the IL15 is mammalian IL2 or IL15, preferably human IL2 or IL15, or a functional variant thereof.

13. The combination according to any of the aforementioned claims, wherein the IL2 comprises a sequence according to SEQ ID No 4, or a functional variant thereof.

14. The combination according to any of claims 1 - 12, wherein the IL15 comprises a sequence according to SEQ ID No 12, or a functional variant thereof.

15. The combination according to any of the aforementioned claims, wherein a fusion protein linker is connecting the binding protein and the inflammatory cytokine part.

16. The combination according to any one of claims 14 - 15, wherein the fusion protein linker
• has a length of between ≥ 1 and ≤ 30 amino acids and/or
• comprises a sequence according to SEQ ID No. 5.

17. The combination according to any of the aforementioned claims, wherein the fusion protein is PEGylated.

18. The combination according to any of the aforementioned claims, wherein the chimeric antigen receptor (CAR) in the T cell comprises 14.G2a-zeta, 14.G2a-BBzeta or 14.G2a-28zeta.

19. The combination according to any one of claims 1 - 18 for use in the treatment of a human or animal subject
• suffering from,
• at risk of developing, and/or
• being diagnosed for
a given pathologic condition.

20. The combination according to claim 19, for use in the therapy of a pathologic condition, wherein the pathologic condition is a neoplastic disease, preferably a solid tumor, in particular a lymphoma, carcinoma, sarcoma, or a leukemia.

21. The combination according to any of the aforementioned claims for use in the therapy of the aforementioned pathologic conditions, wherein the fusion protein and the chimeric antigen receptor (CAR)-T cell are to be administered as concomitant and/or adjunctive therapy.

22. The combination according any of the aforementioned claims for use in the therapy of the aforementioned pathologic conditions, wherein the fusion protein and the chimeric antigen receptor (CAR)-T cell are to be administered as sequential therapy.

## Patentansprüche

1. Kombination mindestens aufweisend
a) ein Fusionsprotein aufweisend
a1) ein Bindeprotein, das ein krebsbezogenes Antigen spezifisch erkennt, und
a2) ein inflammatorisches Zytokin, und
b) eine chimärer Antigenrezeptor (CAR)-T-Zelle, die ein krebsbezogenes Antigen spezifisch erkennt,
wobei das Bindeprotein mindestens ein Mitglied aufweist ausgewählt aus der Gruppe von
• Antikörper,
• modifiziertes Antiköperformat,
• Antikörperderivat oder -fragment, die Zielbindeeigenschaften beibehaltend,
• Antikörper-basiertes Bindeprotein
• Oligopeptidbinder und/oder
• ein Antikörper Mimetikum

2. Kombination nach Anspruch 1, wobei
• das krebsbezogenes Antigen, das vom Bindeprotein erkannt wird, Krebsstroma zugehörig ist und/oder
• die chimärer Antigenrezeptor (CAR)-T-Zelle ein krebsbezogenes Antigen erkennt.

3. Kombination nach Anspruch 1, wobei das krebsbezogene Antigen, das vom Bindeprotein erkannt wird, ein Angiogenesemarker ist.

4. Kombination nach einem der vorherigen Ansprüche, wobei das krebsbezogene Antigen, das vom Bindeprotein erkannt wird, ein Fibronektin, oder eine Splice-Isoform davon, und/oder eine Subdomäne davon ist.

5. Kombination nach einem der vorherigen Ansprüche, wobei das krebsbezogene Antigen, das vom Bindeprotein erkannt wird, die ED_{B}-Domäne von Fibronektin ist.

6. Kombination nach einem der vorherigen Ansprüche, wobei das inflammatorische Zytokin ein Mitglied ausgewählt aus der Gruppe bestehend aus IL2 und IL15 ist.

7. Kombination nach einem der vorherigen Ansprüche, wobei die CAR-T-Zelle Disialogangliosid GD2 erkennt.

8. Kombination nach einem der vorherigen Ansprüche, wobei das Bindeprotein mindestens eine CDR Sequenz vom L19 Antikörper umfasst.

9. Kombination nach einem der vorherigen Ansprüche, wobei das Bindeprotein die Sequenzen nach SEQ ID Nr. 6 bis 11 aufweist.

10. Kombination nach einem der vorherigen Ansprüche, wobei das Bindeprotein mindestens eine V schwere Kette nach SEQ ID Nr. 1 oder mindestens eine V leichte Kette nach SEQ ID Nr. 2 aufweist.

11. Kombination nach einem der vorherigen Ansprüche, wobei die schwere und die leichte Kette durch einen Peptidlinker verbunden sind, wobei der Peptidlinker vorzugweise eine Sequenz nach SEQ ID Nr. 3, oder eine Sequenz mit mindestens 90% Identität zu der Sequenz nach SEQ ID Nr. 3 aufweist.

12. Kombination nach einem der vorherigen Ansprüche, wobei das IL2 oder das IL15 Säuger IL2 oder IL15, vorzugsweise menschliches IL2 oder IL15, oder eine funktionelle Variante davon ist.

13. Kombination nach einem der vorherigen Ansprüche, wobei das IL2 eine Sequenz nach SEQ ID Nr. 4, oder eine funktionelle Variante davon aufweist.

14. Kombination nach einem der Ansprüche 1 - 12, wobei das IL15 eine Sequenz nach SEQ ID Nr. 12, oder eine funktionelle Variante davon aufweist.

15. Kombination nach einem der vorherigen Ansprüche, wobei ein Fusionsproteinlinker das Bindeprotein und das imflammatorische Zytokin verbindet.

16. Kombination nach einem der Ansprüche 14 - 15, wobei der Fusionsproteinlinker
• eine Länge zwischen ≥ 1 und ≤ 30 Aminosäuren hat und/oder
• eine Sequenz nach SEQ ID Nr. 5 aufweist.

17. Kombination nach einem der vorherigen Ansprüche, wobei das Fusionsprotein PEGyliert ist.

18. Kombination nach einem der vorherigen Ansprüche, wobei der chimäre Antigenrezeptor (CAR) in der T-Zelle 14.G2a-zeta, 14.G2a-BBzeta oder 14.G2a-28zeta aufweist.

19. Kombination nach einem der Ansprüche 1 - 18, für die Verwendung in der Behandlung von einem menschlichen oder tierischen Patienten
• leidend unter,
• gefährdet zu entwickeln, und/oder
• diagnostiziert für
einen pathologischen Zustand.

20. Kombination nach Anspruch 19 für die Verwendung in der Therapie von einem pathologischen Zustand, wobei der pathologische Zustand eine neoplastische Krankheit ist, vorzugsweise ein solider Tumor, insbesondere ein Lymphom, Karzinom, Sarkom, oder eine Leukämie.

21. Kombination nach einem der vorherigen Ansprüche für die Verwendung in der Therapie der vorgenannten pathologischen Zustände, wobei das Fusionsprotein und die chimärer Antigenrezeptor (CAR)-T-Zelle als begleitende und/oder adjuvante Therapie zu verabreichen sind.

22. Kombination nach einem der vorherigen Ansprüche für die Verwendung in der Therapie der vorgenannten pathologischen Zustände, wobei das Fusionsprotein und die chimärer Antigenrezeptor (CAR)-T-Zelle als sequentielle Therapie zu verabreichen sind.

## Revendications

1. Combinaison comprenant au moins
a) une protéine de fusion comprenant
a1) une protéine de liaison reconnaissant de manière spécifique un antigène lié à un cancer et
a2) une cytokine inflammatoire, et
b) une cellule à récepteur antigénique chimérique (CAR)-T reconnaissant un antigène lié à un cancer,
dans laquelle la protéine de liaison comprend au moins un élément du groupe sélectionné parmi
• anticorps,
• format d'anticorps modifié,
• fragment ou dérivé d'anticorps conservant des propriétés de liaison de cible
• protéine de liaison à base d'anticorps,
• liant oligopeptidique et/ou
• un mimétique d'anticorps.

2. Combinaison selon la revendication 1, dans laquelle
• l'antigène, lié à un cancer reconnu par la protéine de liaison, est lié à un stroma cancéreux et/ou
• la cellule à récepteur antigénique chimérique (CAR)-T reconnaît un antigène lié à une cellule cancéreuse.

3. Combinaison selon la revendication 1, dans laquelle l'antigène, lié à un cancer reconnu par la protéine de liaison, est un marqueur d'angiogenèse.

4. Combinaison selon l'une quelconque des revendications précédentes, dans laquelle l'antigène, lié à un cancer, reconnu par la protéine de liaison est une fibronectine, ou une isoforme d'épissage de celle-ci, et/ou un sous-domaine de celle-ci.

5. Combinaison selon l'une quelconque des revendications précédentes, dans laquelle l'antigène, lié à un cancer, reconnu par la protéine de liaison est le domaine ED_{B} de fibronectine.

6. Combinaison selon l'une quelconque des revendications précédentes, dans laquelle la cytokine inflammatoire est une sélectionnée dans le groupe constitué par IL2 et IL15.

7. Combinaison selon l'une quelconque des revendications précédentes, dans laquelle le lymphocyte CAR-T reconnaît le disialoganglioside GD2.

8. Combinaison selon l'une quelconque des revendications précédentes, dans laquelle la protéine de liaison contient au moins une séquence CDR de l'anticorps L19.

9. Combinaison selon l'une quelconque des revendications précédentes, dans laquelle la protéine de liaison comprend les séquences selon SEQ ID n° 6 à 11.

10. Combinaison selon l'une quelconque des revendications précédentes, dans laquelle la protéine de liaison comprend au moins une chaîne lourde V selon SEQ ID n° 1 ou au moins une chaîne légère V selon SEQ ID n° 2.

11. Combinaison selon l'une quelconque des revendications précédentes, dans laquelle les chaînes lourde et légère sont reliées par un lieur peptidique, dans laquelle de préférence le lieur peptidique comprend une séquence selon SEQ ID n° 3, ou une séquence ayant une identité d'au moins 90 % avec la séquence selon SEQ ID n° 3.

12. Combinaison selon l'une quelconque des revendications précédentes, dans laquelle l'IL2 ou l'IL15 est une IL2 ou une IL15 mammifère, de préférence une IL2 ou une IL15 humaine, ou une variante fonctionnelle de celle-ci.

13. Combinaison selon l'une quelconque des revendications précédentes, dans laquelle l'IL2 comprend une séquence selon SEQ ID n° 4, ou une variante fonctionnelle de celle-ci.

14. Combinaison selon l'une quelconque des revendications 1 à 12, dans laquelle l'IL 15 comprend une séquence selon SEQ ID n° 12, ou une variante fonctionnelle de celle-ci.

15. Combinaison selon l'une quelconque des revendications précédentes, dans laquelle un lieur protéique de fusion relie la protéine de liaison et la partie cytokine inflammatoire.

16. Combinaison selon l'une quelconque des revendications 14 à 15, dans laquelle le lieur protéique de fusion
• a une longueur comprise entre ≥1 et ≤ 30 acides aminés et/ou
• comprend une séquence selon SEQ ID n° 5.

17. Combinaison selon l'une quelconque des revendications précédentes, dans laquelle la protéine de fusion est PÉGylée.

18. Combinaison selon l'une quelconque des revendications précédentes, dans laquelle le récepteur antigénique chimérique (CAR) dans le lymphocyte T comprend 14.G2a-zêta, 14.G2a-BBzêta ou 14.G2a-28zêta.

19. Combinaison selon l'une quelconque des revendications 1 à 18 pour utilisation dans le traitement d'un sujet humain ou animal
• souffrant de,
• en danger de développement de, et/ou
• étant diagnostiqué pour
un état pathologique donné.

20. Combinaison selon la revendication 19, pour utilisation dans la thérapie d'un état pathologique, dans laquelle l'état pathologique est une maladie néoplasique, de préférence une tumeur solide, en particulier un lymphome, un carcinome, un sarcome, ou une leucémie.

21. Combinaison selon l'une quelconque des revendications précédentes, pour utilisation dans la thérapie des états pathologiques précédemment mentionnés,
dans laquelle la protéine de fusion et la cellule à récepteur antigénique chimérique (CAR)-T doivent être administrées en tant que thérapie concomitante et/ou complémentaire.

22. Combinaison selon l'une quelconque des revendications précédentes pour utilisation dans la thérapie des états pathologiques précédemment mentionnés,
dans laquelle la protéine de fusion et la cellule à récepteur antigénique chimérique (CAR)-T doivent être administrées en tant que thérapie séquentielle.
